# EUROPEAN PATENT APPLICATION

(11) **EP 4 404 109 A1**
(43) Date of publication of application: **24.07.2024**
(21) Application number: 22868484.1
(22) Date of filing: 31.08.2022
(51) Int. Cl.: G06N 20/00, E21B 47/005

(54) **COMPUTING METHOD FOR DETECTING AND ESTIMATING CEMENTING FAULTS IN OIL WELL LININGS BY ACQUIRING ACOUSTIC PROFILING SIGNALS THROUGH THE PRODUCTION TUBING ON THE BASIS OF MACHINE LEARNING AND HIGH-FIDELITY SIMULATIONS**

(30) Priority: 17.09.2021 BR 102021018581
(71) Applicant: Faculdades Católicas, 22451-900 Rio de Janeiro - RJ (BR); Repsol Sinopec Brasil S.A., 22250-040 Rio de Janeiro (BR); Alis Soluções em Engenharia Ltda., 20921-392 Rio de Janeiro (BR)
(72) Inventor: DE CASTRO RIBEIRO, Mateus Gheorghe, 36325-000 Tiradentes, MG (BR); HULTMANN AYALA, Helon Vicente, 22231-180 Rio de Janeiro (BR); CONCI KUBRUSLY, Alan, 22451-070 Rio de Janeiro (BR); BRASIL DE SOUZA, Luis Paulo, 22281-080 Rio de Janeiro (BR); SANTISTEBAN HIDALGO, Juan Andrés, 24754-350 São Gonçalo - RJ (BR); GIRON CAMERINI, Isabel, 24360-030 Niterói - RJ (BR); DE MAGALHÃES CORREIA, Tiago, 24241-001 Niterói - RJ (BR); LEITE CAVALCANTE, Thiago, 20250-000 Rio de Janeiro (BR); RAMOS LOUZADA, Daniel, 22241-020 Rio de Janeiro (BR); GRECO DE SOUSA, Bruno, 22280-100 Rio de Janeiro (BR); GUANDALINI BATISTA, João Humberto, 22621-251 Rio de Janeiro (BR); VALLADARES DE ALMEIDA, Rafael, 20510-100 Rio de Janeiro (BR)
(74) Representative: Wittop Koning, Tom Hugo
(86) International application number: PCT/BR2022/050346
(87) International publication number: WO 2023/039653

(57) **Abstract**

The present invention proposes using high-fidelity digital simulation of waves guided through the production column, together with monitored learning methods, in a totally automated and data-guided approach, to detect the quality of the oil well lining cement through the production tubing. For this purpose, a series of simulations are used in the most usual conditions of cement quality failure to perform predictive modelling based on machine learning. The thus created model can interpret the complex patterns generated from this physical phenomenon, adding value to assist decision-makers in the task of interpreting acoustic profiling data. The final objective is to isolate and identify cement defects in wells, using acoustic waves guided through the production column, which generate signals of difficult interpretation when compared with the cases of simple linings which require, however, highly qualified professional training to carry out a very demanding and error-prone task.

## Description

### FIELD OF APPLICATION

Plug and abandonment (P&A) operations in oil production wells are performed when the well is decommissioned; however, before doing so, it is necessary to inspect the quality of the sealing liner cement through acoustic profiling techniques, which currently require removal of the production column.

Consequently, there is great interest from the petroleum production industry in proposing solutions that allow cement sealing quality evaluations through acoustic profiling methods used in the production column, in order to lower P&A costs by avoiding the removal of the production column.

Correct P&A completion is important for preventing leaks occurring after well abandonment, which may impact the environment and the economic order.

### SUMMARY OF THE INVENTION

In this context, the use of machine learning techniques has been investigated, associated with data on acoustic waves guided through the production column, in order to assist decision-makers with the interpretation of measurements performed under these conditions, which are intensive, highly specialized, and error-prone.

The data on acoustic waves guided through the production column have complex patterns and are thus harder to interpret than a simple lining context.

Assuming the availability of acoustic profiling data from the production column has precise indications of cement faults, supervised machine learning may be used to handle cement quality evaluations, providing monitoring metrics that help decision-makers.

Currently, the use of machine learning in cement quality evaluations lacks the application of algorithms based solely on artificial intelligence.

The current solutions are not based exclusively on statistics and machine learning, relying on estimates of geophysical variables, such as dispersion curves, typically in terms of slow frequency functions.

Constructing methods through only artificial intelligence paradigms would ensure scalable solutions able to adapt to new situations, through an approach based only on measured data, in order to provide continuous support that may be adapted to the decision-making process.

Guided acoustic wave analysis, such as dispersion curves, has recently been used, together with machine learning methods, to solve the problem of estimating cement quality along the production column.

However, these hybrid solutions are based on scatter curves and machine learning, with constraints in the model creation process, whose scope is limited to the use of information in the temporal and spatial frequency field.

In contrast, in the production column profiling context, where dispersion curves demonstrate subtle changes in contexts with massive variations in cement quality, the process of creating predictive models is impacted by not making good use of all the acoustic wave data content, by examining only spectrum content information.

This opens up opportunities for using artificial intelligence methods to solve the cement quality estimation problem, ensuring effective use of all the information in these signals, such as measurements, thus doing away with the need for pre-processing to obtain estimates of geophysical variables.

Furthermore, the model construction data source is a hurdle hampering the implementation of solutions based on machine learning in commercial products.

It is known that cement quality evaluation measurement data are expensive, using acoustic signals, while machine learning methods require significant amounts of experimental data to underpin the feasibility of constructing predictive models.

An alternative to taking field measurements is to perform high-fidelity simulations with experimentally validated numerical models that do not depend on experts to label them for supervised learning processes.

Construction of the supervised learning-based model will allow field data evaluations and may also reuse this information to construct or adapt a new model.

A high-fidelity simulation is a cheaper and more feasible alternative for providing a large database with detailed cement quality information, as required to create predictive models.

However, the state of the art lacks a well-defined method for generating these data, as reflected in the faults most commonly found in the field. This is a relevant step for creating classifiers, because input data quality defines the success of their application.

This invention proposes the use of a high-fidelity numerical simulation of the guided wave phenomenon in the production column, together with supervised learning methods, in a fully automated and data-guided approach.

For this purpose, a set of simulations is adopted, under the most common cement quality fault conditions, in order to proceed with predictive modelling based on machine learning.

Hence, the created model may interpret the complex patterns generated by this physical phenomenon, generating value that assists the decision-maker when interpreting acoustic profiling data.

The purpose is to isolate and identify cement faults in wells, based on acoustic waves guided through the production column and generating signals that are harder to interpret than simple linings, consequently requiring highly qualified professional training for an intensive but error-prone task.

### BACKGROUND ART

The state of the art already encompasses patent document WO 2016187240A1, which proposes a method for analysing cement integrity with acoustic tools, with multiple lining measurements taken through the usual profiling tools, giving rise to dispersion curves that are then compared with patterns, visually establishing which most closely resembles the item under analysis.

The state of the art related to this application considers a context with multiple linings, with the slowness dispersion curves analysed by an expert, who provides a comparison-based subjective analysis of the measurements.

The current state of the art also encompasses patent document WO 2017151834A1 which proposes a well integrity analysis using sonic measurements at depth intervals, using slowness and acoustic signal attenuation data.

This approach identifies the presence of fluids in the outer region, between the lining and the rock formation, through a combination of patterns formed along a contour.

The state of the art also encompasses patent document WO 2016187242A1, which proposes a cement quality analysis method for analysing wells with multiple linings, using classifiers based on machine learning to detect the presence of cement or fluid between the linings and/or between the lining and the rock formation.

The classifiers are constructed according to the estimated attributes of geophysical variables, such as dispersion curves, obtained from measurements made by acoustic profiling tools.

Another source of information is added, together with the classifier response, based on the multimodal inversion that provides cement density and acoustic impedance properties.

The state of the art also encompasses patent document WO 2019084219A1, which addresses the sealing cement quality analysis problem through classifiers based on machine learning. These model inputs are generated from geophysical variables, such as Radon transform slowness estimates or slowness time coherence (Kimball, C., and T. Marzetta, 1984, Semblance processing of borehole acoustic array data: Geophysics, 49, 274-281, doi: 10.1190/1.1441659), where the proposed classifiers are the support vector machines, convolutional and auto-encoder, artificial neural networks, which may be associated with other frequency domain characteristics through mel-cepstral coefficients.

The above-mentioned artificial neural networks are used because they can be automated, together with the process for creating the final classification characteristics, using images obtained from the estimated slowness curves, where the created model outputs are classifications between nominal and fault situations.

The state of the art also encompasses patent document WO 2019236832A1, which proposes unsupervised cement integrity pattern separation through acoustic wave data, where signals measured with acoustic profiling tools are pre-processed through some method that provides you physical data on measurements, such as matrix pencil or slowness time coherence algorithms.

Representing a high-dimensional space, the dispersion curves are reduced through unsupervised learning methods, such as self-organizing maps, auto-encoder (variational) neural networks or generative adversarial networks. The output of this unsupervised step may then be interpreted by a grouping algorithm, such as k-means, with this latter classifier indicating whether a pattern is nominal or faulty.

The developments proposed in patent documents WO 2016187240A1 and WO 2017151834A1 require expert analysis, which may be laborious and time-consuming, due to the large number of patterns to be analysed.

Furthermore, the activity is prone to errors, due to the subtle nature of alterations in the acoustic signals when the tested cement quality varies, particularly with multiple layers.

The developments proposed by patent documents WO 2016187242A1, WO 2019084219A1, and WO 2019236832 propose the creation of classifiers based on machine learning for evaluating cement with input data that depend on pre-processing based on geophysical variables, such as the estimated slowness curve. This limits the scope of application for machine learning methods and fails to make good use of other general parameter extraction methods that could benefit from tool architectures other than the usual options, designed specifically for multiple linings. For example, they might use measurements taken by the tool next to the production column wall, a situation where it would not be possible to apply the same strategy, due to the lack of generalization for methods based on geophysical variables.

Moreover, it is known that the wave pattern changes the slowness curve almost imperceptibly in the production column profiling condition, hampering use of the usual geophysical parameters.

In all the patents analysed above, dispersion curves and slowness curves are compared with signatures of each of the faults. However, the method of obtaining patterns for use as comparisons is not specified, and their corresponding acoustic signals are not specified.

The development proposed in patent document WO 2016187242A1 mentions the creation of models using synthetic data, although without specifying how they are obtained.

The development proposed in patent document WO 2019084219A1 suggests setting up a database for the machine learning-based classifier creation process, using cement evaluation maps and expert interpretation.

This process is too intensive, if not impractical, due to the number of samples generally needed to train models such as those proposed in the aforementioned document (about a million items),also mentioning the possibility of synthetic scenarios, again without specifying how they are obtained.

Another fact disregarded in the analysed patents is the high computing cost of simulations, furthermore, to the need for outlining a strategy that allows the signals to be obtained in a timely manner, as required to create models for implementation in the field.

In the development proposed in patent document WO 2016187240A1, the dispersion and slowness curve data are compared with patterns, without adding any cement quality index generation layers, and evaluations are performed by an expert, through comparison with a known pattern. In addition to overburdening the expert, this prevents automating quality metrics generation tasks, as it depends on this expert.

In the developments proposed in patent documents WO 2016187242A1 and WO 2019084219A1, the classifiers provide classification only between nominal cases and cement quality faults, thus hampering the detection of areas with subtle defects and failing to use fault size information in the data used to create the classifiers.

Fault estimations provide decision-makers with more information, as well as allowing the establishment of metrics and views for interactions with users. Furthermore, cement quality estimates may also be used as fault probability predictors in well sections.

In the developments proposed in patent documents WO 2016187242A1 and WO 2019084219A1, the manner of developing the machine learning model is not specified in detail, thus implying subjective choices when creating this artifact.

### BACKGROUND OF THE INVENTION

The purpose of this invention is to propose a computer-based method for detecting and estimating of cementing faults in oil well linings, through the acquisition of acoustic profiling signals in the production column, machine learning-based and in high-fidelity simulations, then generalizing the machine learning methods in order to maximize use of the information in the measurements, regardless of the nature of the acoustic profiling tool, surmounting the limitation at the state of the art, based only on the use of geophysical variables. This makes it possible not only to encompass the most efficient machine learning methods, but also to explore nonconventional tool architectures.

Moreover, a strategy is proposed for creating machine learning model training databases, using high-fidelity model numerical simulations, thus enabling the development of predictive models that can quantify the severity of faults based on different types of measurements, meaning measured experimentally or in the field, for complex analyses, even when undertaken by experts.

The inference of the predictive model may be interpreted as a smart check of the measurements under analysis with the simulation database, which is a laborious pattern recognition task.

This invention helps provide decision-makers with greater granularity in cement quality analyses, which is usually a tricky and error-prone task.

In general, the method proposed in this invention allows the following advances, compared to the current state of the art:
- Detecting and estimating cement quality based on acoustic measurements along the production column and supervised learning, ensuring greater granularity in diagnoses and providing richer information for decision-makers, in contrast to the binary nominal/fault classification allowed by existing solutions;
- Establishing a process for preparing a representative dataset, based on numerical simulations and used to define classifiers; this was not established with the level of detail required for replication, allowing the generation of a significant number of samples as required to perform supervised learning and then prepare predictive models able to indicate cement quality through acoustic measurements, as mentioned in the previous paragraph;
- Using time domain data, by reducing measurement dimensionality, thus ensuring greater generalization and maximizing the use of measurements in the model development process, in contrast to the exclusive use of geophysical variables with subtle changes in measurements caused by variations in cement quality, when profiling along the production column; this ensures the use of different architecture tools, while also allowing the application of machine learning methods based exclusively on measured data; furthermore, it is less complex, as there is no need to extract dispersion curves, which is a process required by all the methods presented in the literature;
- Minimizing the need for expert intervention in the classifier creation process based on hyperparameter random search and resampling methods, which allows automated construction of predictive models used to detect and estimate cement quality, while the prior art fails to specify ways of obtaining predictive models, specifying only the dataflows for each method and thus allowing subjective choices to interfere with end product quality, which is undesirable; and
- Adding field measurement results to the model, and performing new training based on this process, as soon as new samples are available.

### BRIEF DESCRIPTION OF THE FIGURES

In order to better understand the computer-based for detecting and estimating cementing faults in oil well linings through the acquisition of acoustic profiling signals along the production column, with high-fidelity machine learning-based simulations, reference is made to the Figures appended hereto, wherein:

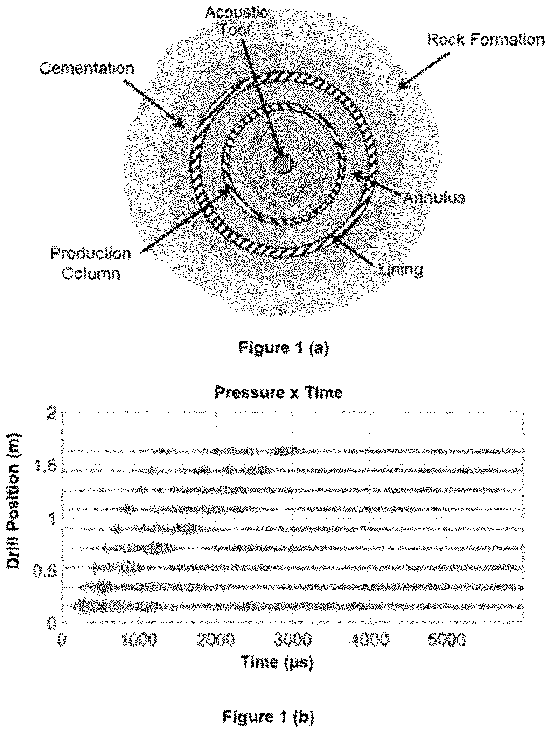

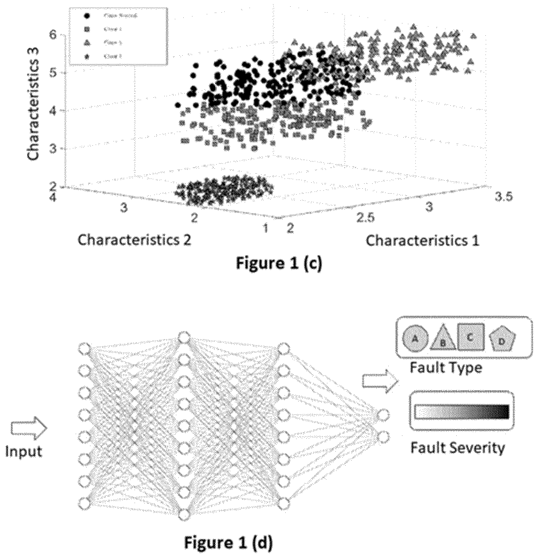

The Figure 1 illustrates (a) the process for detecting and estimating cementing faults in oil well linings through the acquisition of acoustic profiling signals along the production column, (b) the taken measurements that constitute the information matrix, (c) the reduction of dimensionality and prediction variables, (d) the hybrid classification/regression architecture based on supervised learning for inferring and diagnosing predictions of fault types and magnitudes;

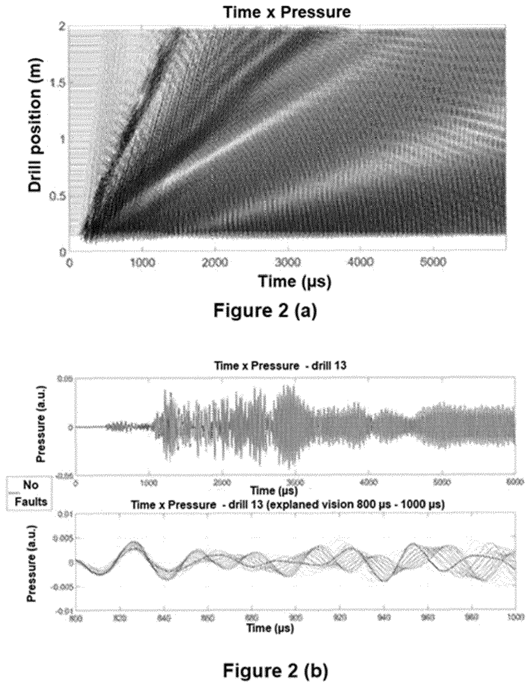

The Figure 2 illustrates examples of measurements obtained at (a) several tool contours through the finite elements method, and (b) varying cement quality in a set of cement fault scenarios within a timeframe and with initial magnification, where it is noted that (a) it is possible to add different measurements the corresponding to assorted tool architectures and (b) that the generated patterns are subtle when cement quality varies, requiring assistance from tools that facilitate interpretation by the decision-maker;

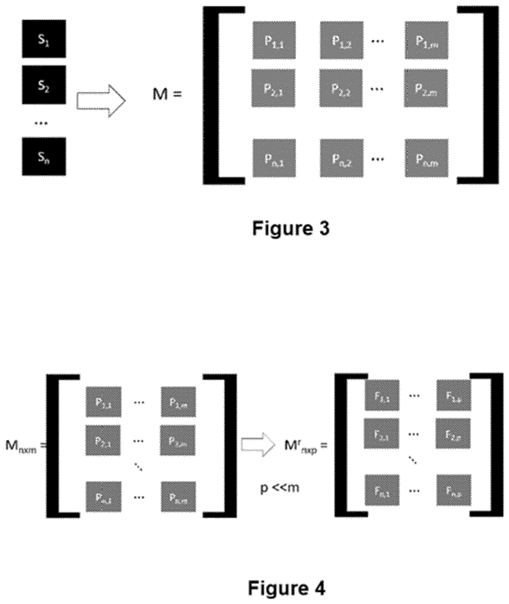

The Figure 3 illustrates the establishment of the information matrix, where Si refers to the simulation of the i-th case and Pi,j denotes the time series of the j-th measurement available in the profiling tool, with all the time series s concatenated in order to create a high-dimensional vector representing the simulated context under analysis, with the matrix consisting of general information that allows aggregation of the available number of measurements, regardless of the profiling tool architecture;
The Figure 4 illustrates the dimensional reduction process, which is intended to provide a reduced information matrix (Mr) based on the information matrix (M), which is a generic process that can handle different types of profiling tool measurements. it also ensures the predictive model inputs are informative but not unnecessarily complex, thus enhancing the chances of success for the predictive model creation process;

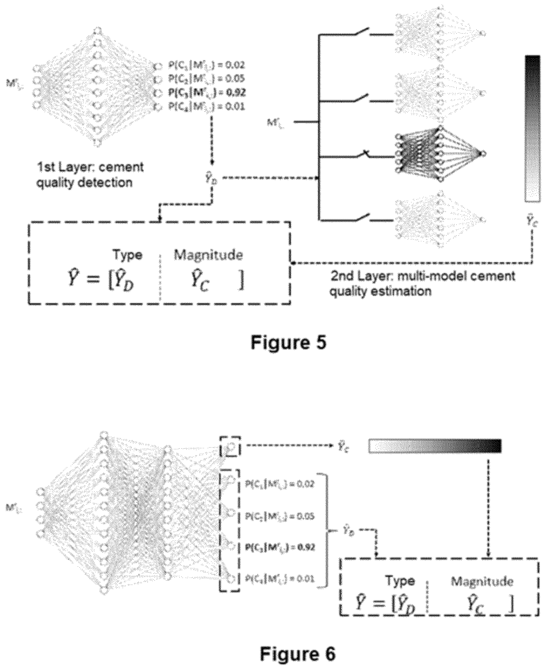

The Figure 5 illustrates the predictive model architecture for detecting and estimating cement quality, in a hierarchical manner, using acoustic profiling along the production column, where the models are represented by neural networks in this example, which may be replaced by other classes of models, with no loss of general applicability for the invention, where the first layer classifies the cement type using information in the i-th line of the reduced information matrix, with an output showing the type of cement fault or if it is nominal; this output may be a probability of belonging to a class, or just category information, with this estimate used to select which fault estimation model will be used to provide the fault estimate, with the final prediction depending on the concatenation of the predictions in the first and second layers, providing the classification and an estimate of the magnitude of a fault; and
The Figure 6 illustrates the cement quality detection and estimation model architecture simultaneously, using acoustic profiling along the production column, which, for the approach under analysis, splits the last layer into neurons producing the classification output, with a SoftMax-type activation function and a neuron producing the linear-type regression output with an activation function, with the final prediction of the fault based on a single model.

### PREFERRED DESCRIPTION OF THE INVENTION

As illustrated in Figure 1, the computer-based method for evaluating oil well sealing cement quality, based on acoustic profiling data acquired along the production column, according to this invention includes; (i) a process for simulating the physical process through high-fidelity models; (ii) generation of data-based cement quality metrics; (iii) construction of the information matrix, used as data stream input; (iv) dimensionality reduction; (v) construction of models with architectures that allow cement quality detection and estimation, based on acoustic profiling.

In general, each of the above-mentioned steps may be summarized as set forth below.

The first step of the method is to construct the numerical simulation database, which is then used to create a predictive model for detecting and estimating cement quality when subject to faults; these simulation data are used for two purposes: the first is to generate the cement quality metrics, according to the fault types most commonly found in the field, thus allowing such faults to be not only isolated, but also quantified, the second underpins the setup of the information matrix, which is composed of all the time-based datasets acquired as responses to sending out excited guided waves through the profiling tool, with the information matrix then broken down through dimensionality reduction methods, since its use with no parameter extraction results in predictive models with poor generalization capabilities, and through dimensionality reduction and the establishment of cement quality metrics, a predictive model is developed through supervised learning that can map the information matrix for cement quality metrics; in this way, the machine learning-based model may be queried with new data acquired either experimentally or through field measurements, in order to provide predictions about cement fault types and magnitudes, with the cement quality detection and estimation model suitable for implementation in a computer-based system, through which decision-makers may include metrics that help them analyse large quantities of acoustic profiling data that hard to interpret.

Set forth below is a detailed explanation of each of the items that compose the invention.

Experimental acoustic profiling measurement data may be acquired through commercial acoustic profiling tools that commonly use monopole, dipole, or quadrupole type sources, without being limited thereto, and these sources may of a different type.

Numerical dates may be acquired through commercial simulation tools, typically using finite element or finite difference methods, without being limited thereto, and may also provide dedicated numerical or analytical-numerical codes.

Due to the general characteristic of the information matrix and the dimensionality reduction process, it is possible to apply the method described below with no loss of generality, and the invention may encompass acoustic profiling situations, regardless of the tool architecture used thereby.

It is also important to emphasize that the method proposed by this invention may be implemented rapidly through open-source scientific computing libraries, being well established and accepted in the academic and industrial worlds (Scikit-learn: Machine Learning in Python, Pedregosa et al., JMLR 12, pp. 2825-2830, 2011; Virtanen, P., Gommers, R., Oliphant, T.E. et al. SciPy 1.0: fundamental algorithms for scientific computing in Python. Nat Methods 17, 261-272 (2020). https://doi.org/10.1038/s41592-019-0686-2; van der Walt, S., Colbert, S. C. & Varoquaux, G. The NumPy array: a structure for efficient numerical computation. Comput. Know. Eng. 13, 22-30 -2011).

The construction of models using machine learning may be based on the supervised paradigm, which requires desired input and output pairs, in order to allow adjustments to the predictive model parameters and consequently to perform the correct mapping between inputs and predictions.

An alternative to setting up a database is to perform numerical simulations based on high-fidelity model, ideally validated experimentally in one or more control cases, which adds advantages to the process as a whole, such as: avoiding the need for an expert to label the measurements; allowing the generation of fault scenarios where experiments are difficult; constructing a database with a set of simulations limited only by computer capacity, and avoiding the need to perform a wide range of experiments that require specific instruments, such as expensive test benches, for example, in order to include test bodies with different conditions that simulate possible different faults and their various levels of severity, while also avoiding expensive field data acquisition, with the required database setup strategy based on the application requirements under examination.

One possibility for this is to use numerical modelling of the problem in question, which can reproduce the acoustic characteristics of the problem, and may be handled through commercial software that uses numerical methods such as, but not limited to, finite element or finite difference methods, while dedicated code modelling is also possible, such as, but not limited to, numerical or analytical-numerical options.

Presented below are the steps for creating the response-based model directly in the time domain, based on guided wave modes excitation.

Once the following modelling framework has been defined, the geometry of the problem must be modelled.

For production column profiling, the geometry of a nominal well is provided by concentric cylindrical layers of the various materials that compose it. From the innermost to the outermost layer, they are fluid in the production column; production column, typically steel; fluid outside the production column; lining, typically steel; cement layer, and rock formation.

Each layer must have its own nominal mechanical constants and defined dimensions. Approaches that provide time domain signals require longitudinal dimension modelling of the model, which must be long enough for all the receivers spaced along the well to be encompassed by the problem.

Furthermore, in order to avoid onboard reflections additional length is required, or absorber simulation techniques may be used to mitigate reflection effects (SHEN, Y.; GIURGIUTIU, V. Effective non-reflective boundary for lamb waves: Theory, finite element implementation, and applications. Wave Motion, 58:22-41, 2015).

Models using mesh discretization must do so by complying with the constraints imposed by the minimum wavelength addressed in the problem (Moser, F., L. J. Jacobs, and J. Qu. 1999. "Modeling elastic wave propagation in waveguides with the finite element method", NDT & and International (Ndt & and International) 225-234; Nandy, A., S. Mullick, S. De, and D. Datta. 2009. "Numerical simulation of ultrasonic wave propagation in a flawed domain", National Seminar & Exhibition on NDE).

Then the excitation source must be imposed, which may be of the monopole, dipole, quadrupole, or other type, and may also be simulated directly by the excitation of a pressure signal in the production column, or on its inner surface, or through the complete modelling of the transmission transducer, whereby the signal to be applied is a signal at an electrical voltage analogous to the signal to be applied to the transmitter in an experiment, and the form in time of the excitation signal may be broad or narrow spectrum.

Due to such excitation, the model can simulate the propagation of acoustic waves guided along the geometry in question, while receivers at longitudinal positions of interest acquire the pressure signal directly in the time domain, as in an experimental setup.

Similar to generation, the receiving transducers may be simply an acoustic pressure read at pre-defined positions, or modelled completely, where the received signal corresponds to the electrical voltage read at the terminals, as in an experimental setup.

Signals in non-nominal situations, such as when faults are present, can be easily inserted into the model, which may then be run again in order to provide a new time series reflecting the simulated fault condition.

A wide collection of signals in various fault conditions may be simulated efficiently by using adequate computerized resources available on the market.

Several kinds of faults may be simulated, such as, but not limited to, internal cement tubing type faults; faults in cement adhesion to lining surfaces or to rock formation surfaces, non-nominal cement quality; and well eccentricity, among others (0sterb0, K. 2014." Cement Bond Evaluation", MASTER'S THESIS Department of Mechanical and Structural Engineering and Materials Science, University of Stavanger, Norway; Vimal Saxena, Michel Krief, Ludmila Adam, "Handbook of Borehole Acoustics and Rock Physics for Reservoir Characterization", 2018; Cameron, I., "SPE Back to Basics - Bond Log Theory and Interpretation", 2013; Hayden, R., Russell., C., "Cases Studies in Evaluation of Cement with Wireline Logs in a Deep Water Environment," Society of Petrophysicists and Well-Log Analysts, 2011, Liu, H. 2017, "Principles and Applications of Well Logging," 237-269).

Such faults may be simulated at several severity conditions, by varying the tubing thickness, for example, or non-nominal cement constants.

Models and faults with axial symmetry may be simulated in two-dimensional models in axisymmetry, at lower computing costs than three-dimensional models.

Figure 2 illustrates the acoustic pressure time series acquired for a simulated case study, representing measurements at different contours along the tool.

It should be noted that the approach presented above, based on high-fidelity models, can offset some of the technical faults diagnosed above at the current state of the art. They include the absence of any need for subjective analysis by an expert for classifying a specific condition, as they are obtained through the absence/presence/level of severity, and then have a natural label with this approach underpinning the setup of a huge database, with no need for expensive test benches, limited by only the available computer resources. Finally, there is no problem with response granularity, as possible non-nominal cases may be simulated with arbitrary variations in their generation parameters.

From simulated cases, it is possible to establish output variables for the predictive model and, based on the measured acoustic signals, the predictive model established through supervised learning can distinguish between nominal and fault types, in addition to estimating fault magnitude.

Information on fault magnitude is important, because it ensures the granularity required for detailed diagnoses of lining cement quality.

Consequently, this invention proposes the use of a two-metric cement quality dataset, namely: (i) fault type; and (ii) fault magnitude.

The use of both metrics impacts the model's construction process, as they must be treated differently during the creation process.

[In order to detect the fault, a classification model may be used with SoftMax-type outputs, but not limited to this example, whose outputs reflect the probability of belonging to a specific class; to estimate its magnitude, any regression method may be used.

These options are explored and described in detail below.

According to the setup process for the simulation database, it is possible to establish physical parameters that, when varied, represent a specific fault condition in the system.

Consequently, in order to obtain the output variable of the fault type for supervised learning, all it is needed is to relate the simulated fault type to its respective class, and then, if all the physical parameters are described by a closed dataset, the limit of this constraint maybe used to map the severity metrics directly.

These choices and definitions must use information on the simulation cases as defined above.

The simulation or experimental data acquired to construct or evaluate the model, an information matrix maybe set up with all the measurements for each situation to be analysed, in order to allow treatment with dimensionality reduction techniques, being simulated, with situations for inclusion in the model creation database being simulated, measured in the field, or experimentally.

For each of these situations, a line is reserved in the information matrix for receiving the concatenated values of the time series for each profiling tool transducer receiver; consequently, the information matrix stores all the acoustic pressure measurement time series performed in the tool, for all measured or simulated situations.

The information matrix may also be created for situations where cement quality must be evaluated, these data may be simulated, measured experimentally, or measured in the field, adopting exactly the same process. The only constraint is that tool architecture data must correspond to the contacts used to create the model, in addition to evaluating cement quality during tool use.

Figure 3 illustrates the process for creating the M information matrix, when n simulations are run, and m measurements obtained at different points of the profiling tool.

Constructed as specified above, the information matrix shall have high dimensionality if applied in the supervised learning process, and its number of columns is proportional to the number of transducers and the sampling rate, which is related to the time required for the propagation of the various guided wave modes related to the problem under examination, for appearing in the acquired signals, whereby the number of sampled points may easily top exceed tens of thousands.

As the number of samples needed to estimate a function with arbitrary precision increases exponentially with the number of inputs (curse of dimensionality) (Bellman, Richard." E. 1957. Dynamic Programming." Princeton University Press. Bellman Dynamic Programming I957 (1957): 151.), it is generally necessary to reduce machine learning input data dimensionality, in order to streamline the model construction process.

This invention adopts a purely machine-learning-based approach, using statistics for dimensionality reduction, where the number of columns in the information matrix is reduced, in order to ensure feasible model construction architectures.

Indeed, as measurements at different contours have a certain correlation, it is expected all measured data can be summarized in a limited dataset, generally of considerably smaller dimensions than that in the information matrix.

Any dimensionality reduction method is applicable to the problem in question, such as, but not limited to, breakdown by singular values, principal component analysis, independent component analysis, and factorial analysis.

Furthermore, manifold learning methods may be used, such as, but not limited to, multidimensional scaling, isomaps, and locally linear embedding (Tenenbaum, J. B., De Silva, V., & Langford, J. C. (2000)." A global geometric framework for nonlinear dimensionality reduction", Science 290{500)I 2319-2323; Roweis, S. T., & Saul, L. K. (2000)." Nonlinear dimensionality reduction by locally linear embedding", Science, 290(5500), 2323-2326; Borg, I., & Groenen, P. J. (2005), "Modern multidimensional scaling: Theory and applications," Springer Science & Business Media; Maaten, L. V. D., & Hinton, G. (2008). Visualizing data using t-SNE." Journal of Machine Learning Research", 9(Nov), 2579-2605).

Principal component analysis (Gareth James, Daniela Witten, Trevor Hastie, Robert Tibshirani." An Introduction to Statistical Learning: with Applications in R", New York: Springer, 2013) may be thought of as the dimensionality reduction of any matrix, and may be performed in a manner whereby singular value approximation is sufficient to represent the matrix through the sum of unit rank matrices.

Strategies may be adopted for defining how many singular values will be present in the reduced dimensionality matrix, which would make it possible to automate the reduction process as a whole.

The explained variance method (Brunton, S., & Kutz, J. (2019), "Data-Driven Science and Engineering: Machine Learning, Dynamical Systems, and Control" Cambridge: Cambridge University Press, doi:10.1017/9781108380690) may be defined in a manner that arbitrates approximation precision by singular values, when they have a good signal-to-noise ratio.

Another option is to use the Gavish and Donoho criteria (M. Gavish and D. L. Donoho, "The Optimal Hard Threshold for Singular Values is $4/\sqrt {3}$," in IEEE Transactions on Information Theory, vol. 60, N° 8, pp. 5040-5053, Aug. 2014), calculated on the information matrix dimension and the magnitude of the noise corrupting the measurements, alternatively offering manual selection, according to the maximum number accepted for constructing the model from the supervised learning.

Whatever the information matrix transformation method, completion of this dimensionality reduction step results in a reduced-order information matrix with the desired outputs ready for the supervised learning process.

Figure 4 illustrates the dimensionality reduction process, as well as the notation used in the next steps of the method.

With the reduced dimensionality information matrix and the desired output, which consists of the fault type and severity, represented respectively by discrete and continuous variables, it is finally possible to perform the model creation process.

The final model architecture may be established in two ways, one is illustrated in Figure 5, showing that the task of detecting and estimating fault magnitude is split into two, using a multi-model approach, issuing predictions based on the reduced information matrix for the class to which a level belongs; In other words, nominal cement situations and other fault situations that are more relevant to the matter in question, with such predictions also based on models with SoftMax type outputs, such as multilayer neural networks that may be interpreted as the probability of belonging to a specific situation, or models that define only the class to which the level belongs, such as decision trees, with any approach being valid; they may be tested in order to infer the best model adherence to the dataset used.

In the other, each type of fault or nominal cement situation has its own respective regression model that maps the inputs in the reduced information matrix by fault severity. It is important to note that the step of creating several models and running them according to the estimated detection, is performed in order to separate the cement quality estimation problems, and consequently streamlining this task by reducing the constraints that would exist in a model having to estimate all situations concomitantly.

The advantage of this tiered approach is to separate the problems and solve them one at a time. However, the classification/regression problem may also be solved at the same time, through the information matrix.

The second possibility means adopting the process illustrated in Figure 6. With this approach, both the regression and classification outputs benefit from the same model construction training (Chen, J., Cheng, L., Yang, X., Liang, J., Quan, B., & Li, S. (2019, February), "Joint learning with both classification and regression models for age prediction", In Journal of Physics: Conference Series (Vol. 1168, No. 3, p. 032016). IOP Publishing), whereby the output is thus simultaneous and encompasses both fault detection and quantification. However, both the simultaneous and the tiered forms have the same outcome and purpose: cement quality detection and estimation, using acoustic profiling through the drilling column.

Model creation may be automated by testing several architectures through resampling techniques and random testing of hyperparameters (James Bergstra and Yoshua Bengio. 2012. "Random search for hyperparameter optimization", J. Mach. Learn. Res. 13, null (February 2012), 281-305), in order to ensure the generalization required by the predictive model.

Despite making more intensive use of computer resources, this process allows statistical analyses, and with minimal bias in the choice of architectures and their hyperparameters. In order to evaluate each model class for classification and regression problems in cement quality detection and estimation respectively, a random hyperparameter search is run, within the limits indicated for each model architecture and using K-fold cross-validation in order to evaluate this possibility statistically.

At the end of the hyperparameter search and resampling process, it is possible to use all the training data to create the production model, with a definition of the optimal architecture and model class.

The proposed approach to constructing a predictive model for detecting and estimating cement quality allows the model to be created offline for later use.

Once its architecture and hyperparameters are defined through random search and resampling steps, the model may be constantly updated, according to new field samples that are annotated by an expert through a specific user interface, ensuring its adaptation to new conditions and expert interpretations, in a continuous upgrade process that repeats the sampling process as soon as new samples are available.

The predictive model may be run on a computing platform and added to an oil production well acoustic profiling interface.

By adding the outputs prepared by the proposed model, the expert will receive supervised machine learning information on diagnoses, also based on data from several situations of interest, thereby assisting with the decision-making process.

## Claims

1. Computer-based method for evaluating oil well sealing cement quality, based on acoustic profiling data acquired through the production column, **characterized in that** it comprises:
(i) simulation of the physical process through high-fidelity models;
(ii) generation of cement quality metrics based on measured data;
(iii) construction of the information matrix, used as data stream input;
(iv) dimensionality reduction;
(v) construction of models with architectures that allow cement quality detection and estimation, and
(vi) through the construction of the supervised learning-based model, evaluate field data, which are reused to construct a new model through the adaptation thereof.

2. Method according to claim 1, **characterized in that** once its architecture and hyperparameters are defined, through random search and resampling operations, the model may be constantly updated with new field samples, ensuring its adaptation to new conditions and interpretations.

3. Method according to claim 2, **characterized in that** when updating the model, the resampling process may be repeated as soon as the new samples are available.

4. Method according to any one of the previous claims, **characterized in that** it predicts the construction of the numerical simulation database, used to create a predictive model able to detect and estimate cement quality when subject to faults.

5. Method according to any one of the previous claims, **characterized in that** the simulation data generate the cement quality metrics, according to the fault types most commonly found in the field, in order to allow not only the isolation of such faults, but also their quantification.

6. Method according to any one of the previous claims, **characterized in that** the simulation data allow the establishment of the information matrix, composed of all the time series obtained in response to excitation by guided waves through the profiling tool.

7. Method according to any one of the previous claims, **characterized in that** the information matrix may be broken down through dimensionality reduction methods.

8. Method according to any one of the previous claims, **characterized in that** the reduction in dimensionality and the establishment of cement quality metrics, result in a predictive model created from the supervised learning, which can map the reduced information matrix for the cement quality metrics.

9. Method according to any one of the previous claims, **characterized in that** the machine learning-based model may be quail queried, with new data obtained through field measurements, in order to provide predictions about cement fault types and magnitudes.

10. Method according to any one of the previous claims, **characterized in that** the cement quality detection and estimation model may be implemented in a computer-based system, whereby decision-makers may include metrics that help them analyse acoustic profiling data.

11. Method according to any one of the previous claims, **characterized in that** experimental acoustic profiling measurement data may be obtained through commercial acoustic profiling tools that use common monopole, dipole, or quadrupole type sources, without being limited thereto, and these sources may also be of another type.

12. Method according to any one of the previous claims, **characterized in that** field data may be obtained through commercial simulation tools, typically using finite element or finite difference methods, without being limited thereto, and may also come from dedicated numeric or analytical-numeric codes.

13. Method according to any one of the previous claims, **characterized in that** the construction of models using machine learning may be based on the supervised paradigm, which requires desired input and output pairs, in order to allow adjustments to the predictive model parameters and perform the correct mapping between inputs and predictions.

14. Method according to any one of the previous claims, **characterized in that** the database may be generated by performing numerical simulations based on an experimentally validated high-fidelity model, in one or more control situations.

15. Method according to any one of the previous claims, **characterized in that** numerical modelling able to reproduce acoustic characteristics may be handled through commercial software, using finite element or finite difference methods, but not limited thereto, or by numerical or analytical-numerical codes, but not limited thereto.

16. Method according to any one of the previous claims, **characterized in that** models using mesh discretization must do so in compliance with the constraints imposed by the minimum wavelength treated.

17. Method according to any one of the previous claims, **characterized in that** the excitation source may be monopole, dipole, quadrupole, or some other type, and may also be simulated directly by the excitation of a pressure signal in the production column, or on its inner surface, or through complete transmission transducer modelling, in which case a signal is applied at electrical voltage similar to the signal to be applied in the transmitter, and the shape in time of the excitation signal may be of broad or narrow spectrum.

18. Method according to any one of the previous claims, **characterized in that** due to such excitation, the model can simulate the propagation of acoustic waves guided along the geometry in question and receivers, at longitudinal positions of interest, acquiring pressure signals directly in the time domain.

19. Method according to any one of the previous claims, **characterized in that** similar to generation, the receiving transducers may be simply an acoustic pressure read at predefined positions, or modelled altogether, whereby the received signal corresponds to the electrical voltage read at its terminals.

20. Method according to any one of the previous claims, **characterized in that** signals in non-nominal situations, such as in the presence of faults, may be easily inserted into the model, which may then be run again in order to provide a new time series for the simulated fault condition.

21. Method according to any one of the previous claims, **characterized in that** a vast collection of signals at various fault conditions may be simulated efficiently, using adequate computerized resources that are available on the market.

22. Method according to any one of the previous claims, **characterized in that** various kinds of faults may be simulated, such as, but not limited to, internal cement type tubing faults; cement adhesion faults to the lining surface or the rock formation surface, non-nominal cement quality, and well eccentricity, among others.

23. Method according to any one of the previous claims, **characterized in that** such faults may be simulated at several severity levels by varying the tubing thickness, for example, or non-nominal cement constants.

24. Method according to any one of the previous claims, **characterized in that** models and faults with axial symmetry may be simulated in two-dimensional models in axisymmetry.

25. Method according to any one of the previous claims, **characterized in that** it is possible to create output variables for the predictive model from the simulated situations and, based on measured acoustic signals, the predictive model created through supervised learning may also distinguish between nominal and fault types, and also estimate fault magnitude.

26. Method according to any one of the previous claims, **characterized in that** the fault magnitude information ensures the granularity needed for detailed diagnoses of lining cement quality.

27. Method according to any one of the previous claims, **characterized in that** it is possible to use a classification model with SoftMax type outputs, but not limited thereto, indicating the probability of belonging to a specific class, and, in order to estimate its magnitude, a classification model with SoftMax type outputs may be used, but not limited thereto, which presents the probability of belonging to a given class, and, in order to estimate its magnitude, it is possible to use a classification model with outputs of the SoftMax type, but not limited to it, which indicates the probability of belonging to a specific class; in order to estimate its magnitude, a classification model may be used, with the possibility of using any regression method.

28. Method according to any one of the previous claims, **characterized in that** according to the simulation database setup process, it is possible to establish physical parameters that, when varied, represent a specific fault condition in the system and, in order to obtain the supervised learning output variable for such fault type, the simulated fault type is simply linked to its respective class; and if all the physical parameters are described by a closed dataset, the threshold of this constraint is used for direct mapping of the severity metrics.

29. Method according to any one of the previous claims, **characterized in that** it is possible to set up ducting an information matrix with all as measurements for each situation to be analysed, and allowing treatment with dimensionality reduction techniques, the situations to be included in the database being simulated or experimentally measured, for the creation of the model. A line of the information matrix is reserved for each one of these situations, where the concatenation values for each time series will be entered, for each profiling tool transducer receiver, in order to store all the acoustic pressure time series of measurements performed by the tool, in all measured or simulated situations.

30. Method according to any one of the previous claims, **characterized in that** based on machine learning and dimensionality reduction statistics, the number of columns in the information matrix may be reduced in order to allow model architectures whose construction is feasible.

31. Method according to any one of the previous claims, **characterized in that** it is possible to adopt strategies for defining how many singular values will be included in the reduced dimensionality matrix, for automating the reduction process as a whole.

32. Method according to any one of the previous claims, **characterized in that** the final model architecture may separate fault magnitude detection and estimation, with predictions based on the reduced information matrix of the nominal cement situation class, and with other fault situations that are more relevant, drawn from the models with output interpreted as the probability of belonging to a specific situation, or models that define only the class to which the level belongs, such as decision trees.

33. Method according to any one of the previous claims, **characterized in that** the final model architecture alternatively has its own respective regression model for each type of fault situation or nominal cement, which maps the inputs in the reduced information matrix for fault severity.

34. Method according to any one of the previous claims, **characterized in that** the final model architecture may solve the classification/regression problem at the same time, using the information matrix as input.

35. Method according to any one of the previous claims, **characterized in that** the predictive model may be run on a computer platform and added to an oil well acoustic profiling interface.
